# EUROPEAN PATENT SPECIFICATION

(11) **EP 2 750 637 B1**
(45) Date of publication and mention of the grant of the patent: **05.10.2016**
(21) Application number: 12841154.3
(22) Date of filing: 19.10.2012
(51) Int. Cl.: A61F 5/01, A43B 7/20

(54) **AN ANKLE-FOOT ORTHOSIS ELEMENT AND A MANUFACTURING METHOD THEREFOR**
KNÖCHEL-FUSS-ORTHESEELEMENT UND HERSTELLUNGSVERFAHREN DAFÜR
ÉLÉMENT D'ORTHÈSE POUR LA CHEVILLE ET LE PIED ET PROCÉDÉ DE FABRICATION

(30) Priority: 21.10.2011 SE 11509775 T
(43) Date of publication of application: 09.07.2014
(73) Proprietor: Flexbrace International AB, 23622 Höllviken (SE)
(72) Inventor: FALKENMAN, Lars, S-21145 Malmö (SE); GUNDMARK, Glenn-Olof, S-236 31 Höllviken (SE)
(74) Representative: KIPA AB
(86) International application number: PCT/SE2012/051125
(87) International publication number: WO 2013/058706

(56) References cited:
- EP-A1- 1 857 005
- WO-A1-02/00052
- WO-A1-2005/097014
- US-A- 4 523 394
- US-A- 5 759 168
- US-A1- 2005 235 526
- US-A1- 2006 270 958
- US-B2- 6 945 947

## Description

### Field of the Invention

This disclosure pertains in general to the field of footwear and more particularly to orthotic footwear and even more particularly to an ankle-foot orthosis element.

### Background of the Invention

Dropping of the front of the foot may be caused by weakness or paralysis of the anterior muscles of the lower leg. Foot-drop results in what is called a steppage gait, in which the advancing foot is lifted high in order for the toes to clear the ground. Foot drop can be due to a number of conditions including injury to the muscles that dorsiflex the foot or to the nerves of those muscles, a neuropathy, multiple sclerosis, a stroke, drug toxicity, and diabetes. One way of treating this condition is to use an ankle-foot orthosis (AFO).

An ankle-foot orthosis is a brace, usually of plastic or a carbon-based material, worn on the lower leg and foot to support the ankle, hold the foot and ankle in the correct position, and correct foot-drop. AFO's are also known as foot-drop braces or drop-foot braces.

Using a proper AFO prevents the wearer from dragging the foot and enables the wearer to walk extended intervals without any symptoms of fatigue or of being tired. When walking, the wearer does not have to worry about tripping or falling because of the foot drop.

AFO's usually comprise a foot plate, custom-made and specially adapted to the form of the user's foot, and a supporting strut for fixing the ankle, foot and lower limb of the user in a desired position. For use, an AFO is usually assembled with a rather heavy and stable shoe, like an athletic shoe or a walking boot supporting the orthosis.

This results in a rather large assembly, and putting on the assembly may be experienced as uncomfortable as it requires a number of steps, and the orthosis has to be specially adapted to the foot of the user, so that the user is comfortable with the orthosis.

From WO2007/106017, a below-knee orthosis is known. This below-knee orthosis can be used for patients with drop-foot. However, a disadvantage with this kind of orthosis is that the supporting elements are behind the ankle. This makes it difficult to walk and run normally, although it enables walking. Furthermore, this kind of orthosis does not provide any stability in a lateral direction for preventing twisting the foot. Neither does it provide any stability in any front-rear direction. Furthermore, it either needs to be integrated with a shoe or it may slide or move in relation to the shoe.

From US2009/0076428 A1, an ankle brace is known. This kind of orthosis does not provide any stability in a lateral direction for preventing twisting of the foot. Neither does it provide any stability in any front-rear direction. Furthermore, it either needs to be integrated with a shoe or it may slide or move in relation to the shoe.

From SE528818 C2, an ankle foot orthosis is known. However, an ankle foot orthosis as the one described in this document may move or slide in relation to a shoe if it used in/together with the shoe.
From US2006/270958 A1, an ankle foot orthosis is known. However, an ankle foot orthosis as the one described in this document may move or slide in relation to a shoe if it used in/together with the shoe.
Thus, there is a need for an ankle-foot orthosis, which provides stability sideways, i.e. in a lateral direction, as well as in a front-rear direction.
There is also a need for an ankle-foot orthosis, which does not move or slide in relation to the shoe.
Furthermore, there are orthosis used in various sports. The orthoses used for sports can e.g. be used for prevention of injuries, but also for athletes that have been injured and with the help of an ankle-foot orthosis may be able to return to doing sports earlier, after an injury, than if they had not used the ankle-foot orthosis.
Some prior art ankle-foot orthoses have been used for sports. However the prior art ankle-foot orthosis are generally not lightweight, and not anatomically shaped for a comfortable fit of the foot and the leg in the ankle-foot orthosis.
Furthermore, the prior art orthosis used for sports do neither provide for stability in a lateral direction nor for stability in any front-rear direction. In addition these prior art orthoses may slide or move in relation to the shoe they are in unless integrated with the shoe.
Thus, there is a need for an anatomically shaped ankle-foot orthosis for sport purposes. There is also a need for an ankle-foot orthosis for sport purposes, which provides stability laterally as well as in a front-rear direction and which does not slide or move in relation to the shoe.

### Summary of the Invention

Accordingly, embodiments of the present disclosure preferably seek to mitigate, alleviate or eliminate one or more deficiencies, disadvantages or issues in the art, such as the above-identified, singly or in any combination by providing an ankle-foot orthosis, a manufacturing method therefor according to the appended patent claims.
One disadvantage with prior art ankle-foot orthoses is that they do not provide for an athlete to comfortably exercise a sport or enable comfortable walks or an active life for a oatient.
According to one aspect of the disclosure, an ankle-foot orthosis element is provided, according to claim 1, and wherein the base plate element comprises a concave shape for fixation of the ankle-foot orthosis element in a foot wear, such as a shoe or a sport shoe.
According to another aspect of the disclosure, a method of manufacture of an ankle-foot orthosis element is provided, according to claim 11.

Further embodiments of the disclosure are defined in the dependent claims, wherein features for the second aspect of the disclosure are as for the first aspect mutatis mutandis.
Some embodiments of the disclosure avoids a custom-made footwear.

Some embodiments provide for an ankle-foot orthosis, which is easily manufactured.

Some embodiments provide for a high durability, high strength, light weight, and a long time of comfortable use.
Some embodiments provide an ankle-foot orthosis, which is easy to use and/or easy to don and doff.
Some embodiments provide for fixation of the ankle-foot orthosis element in a foot wear, so that the ankle-foot orthosis element will not slide or move in relation to the foot wear and/or stabilization of the ankle-foot orthosis in lateral direction/front-rear direction.

Some embodiments provide for an optimized concave section, thereby providing an ankle-foot orthosis element, which is comfortable and enjoyable for the wearer, since it does not move in relation to the shoe used.
Some embodiments provide for that the straps can be tightened and/or operated on either side of the ankle-foot orthosis element.
Some embodiments provide for simplified and/or cost-effective manufacturing and an increased durability of the ankle-foot orthosis.
Some embodiments provide for reduction of bacteria.

Some embodiments provide for a light ankle-foot orthosis.

Some embodiments provides stronger support at the lower end of the outer supporting element and/or a more anatomically shaped orthosis, thereby providing for a more comfortable fit of the foot.
Some embodiments provide for an ankle-foot orthosis, which can be put on very easily and in a short time.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the disclosure are capable of will be apparent and elucidated from the following description of embodiments of the present disclosure, reference being made to the accompanying drawings, in which:
Fig. 1 is a front view of the ankle-foot orthosis;
Fig. 2 is a lateral view, from the right side, of the ankle-foot orthosis;
Fig. 3 is a rear view of the ankle-foot orthosis;
Fig. 4 is a lateral view, from the left side, of the ankle-foot orthosis;
Fig. 5 is a top view of the ankle-foot orthosis;
Fig. 6 is a view from below of the ankle-foot orthosis; and
Fig. 7 is a perspective view, from above at an angle, of the ankle-foot orthosis.

### Description of embodiments

Specific embodiments of the disclosure will now be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to an ankle-foot orthosis. However, it will be appreciated that the disclosure is not limited to this application but may be applied to many other applications, including footwear.

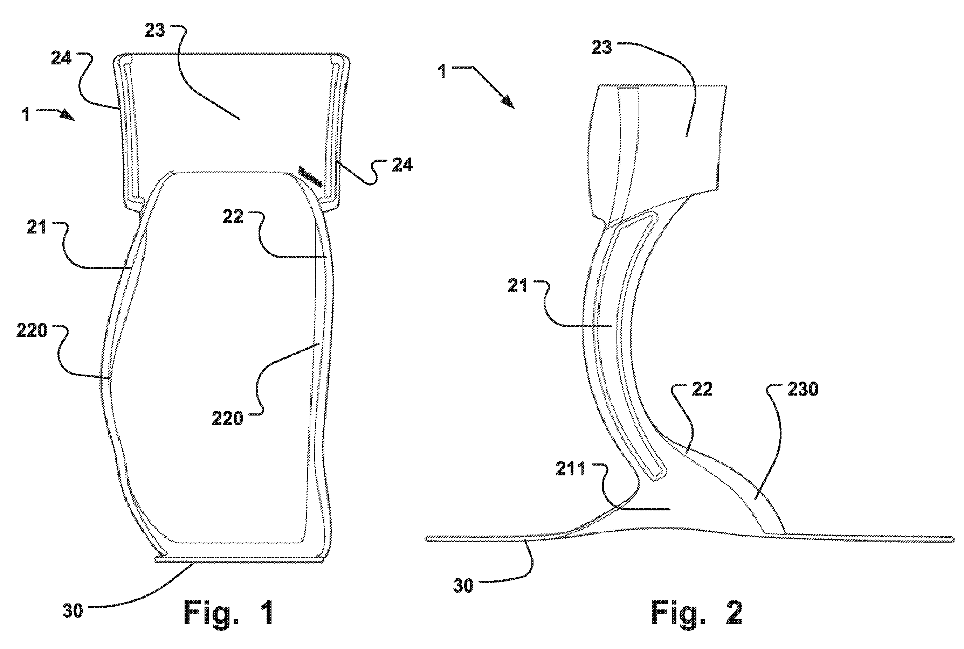

Fig. 1 illustrates the ankle-foot orthosis 1. In this figure different elements of the ankle-foot orthosis 1, such as a base plate element 30 can be seen. The base plate element 30 is connected to at least one supporting element 21, 22 and the at least one supporting element 21, 22 is connected to a top section 23. The supporting element 21 is an inner supporting element intended to be on the inner side of a person's leg, i.e. the side of the leg closest to the other leg, and the supporting element 22 is an outer supporting element intended to be on the outer side of a person's leg. Furthermore, the top section 23 is provided with at least one and preferably two holders 24, for holding fastening means, i.e. a limb fastening member, such as a strap.

When pressure is applied to prior art braces from behind, they are bended forward, i.e. the top portion of the brace is bended forward. Thus, the prior art braces do not provide sufficient stabilization in a front-rear direction. In the present disclosure, each supporting element 21, 22 have a front portion further away from a centre axis running from the rear to the front of the ankle-foot orthosis than a rear portion for at least part of the length of the supporting elements 21, 22, i.e. the supporting elements 21, 22 are angled in at least one portion, such as an intermediate portion 220, in a direction away from, and outwards from an axis of a front-rear direction. Thus, the ankle-foot orthosis opens outwards, i.e. the supporting elements 21, 22 are moving further away from the centre of the ankle-foot orthosis and the top section 23 is lowered, but not bended, when pressure is applied to the top section 23 from behind. Thus, stabilization in a front-rear direction is provided. Furthermore, this way, the top section 23 follows the tibia better than if the top section 23 had been bent.

Fig. 2 is a lateral view, from the right side, of the ankle-foot orthosis 1. In this figure the base plate element 30 together with the supporting elements 21, 22 and the top section 23 can be seen. Furthermore, a lower portion 211 of the inner supporting element 21 and a lower portion 230 of the outer supporting element 22 can be seen. It can also be seen that the lower portion 230 is larger than lower portion 211, i.e. it is extended towards the front. Lower portion 230 may also extend further to the front than the lower portion 211. The positioning of the supporting elements 21, 22 is advantageous, since these elements are preferably positioned in front of the ankles, whereas prior art orthoses have support further to the rear. Thus, a more anatomical shape of the ankle-foot orthosis can be provided.

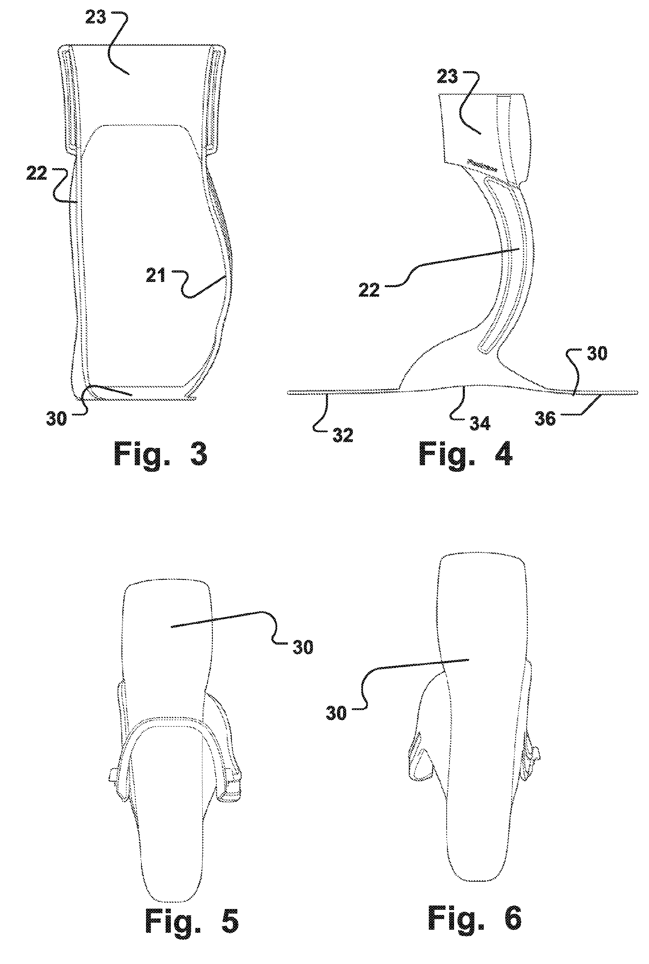

Fig. 3 is a rear view of the ankle-foot orthosis 1 and Fig. 4 is a lateral view, from the left side, of the ankle-foot orthosis 1. Fig. 5 is a top view of the ankle-foot orthosis 1 and Fig. 6 is a view from below of the ankle-foot orthosis 1.

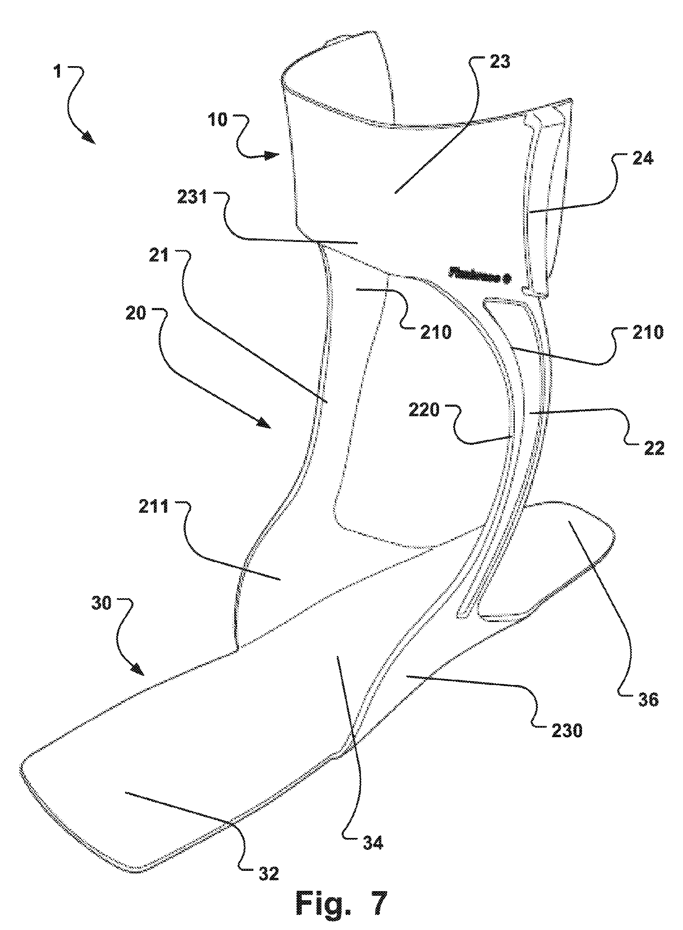

In one embodiment illustrated in Fig. 7, the ankle-foot orthosis 1 comprises an upper section 10, an intermediate section 20 and a base plate element 30. The base plate element 30 of the ankle-foot orthosis comprises a front section 32, which is oriented in the direction of the toes of a wearer of the ankle-foot orthosis 1. The middle section 34 of the lower section 30 of the ankle-foot orthosis 1 is in use oriented approximately at the middle of the wearer's foot sole, and the rear section 36 of the lower section 30 of the ankle-foot orthosis 1 is oriented towards the heel of the wearer. The middle section 34 connects the front section 32 with the rear section 36.

Referring again to fig. 4, it can be seen that the base plate element 30 has a concave shape. The concave shape is for fixation of the ankle-foot orthosis in a footwear, such as a shoe or a sport shoe, as will be described below. Figure 4 shows the base plate element 30 with its front section 32, its middle section 34 and its rear section 36, and it can be seen that the front and the rear sections 32, 36 are substantially flat. However, the middle section 34 is a concave section, i.e. the middle section 34 is bow-shaped with the highest point somewhere in the middle of the section. The concave shape of the middle section 34 is advantageous, since it enables fixation of the ankle-foot orthosis 1 in a footwear. The concave shape provides for that the ankle-foot orthosis does not slide or move in relation to the footwear, since when a user puts his/her foot in the ankle-foot orthosis 1, the foot will press down the concave shape and the ankle-foot orthosis 1 will thereby be unable to move or slide in relation to the footwear. Thus, the ankle-foot orthosis does not need to be integrated with a shoe. Furthermore, when the concave middle section 34 is pressed down, the ankle-foot orthosis 1 is tightened around ankles and the supporting elements 21, 22 are moved closer to the leg, and therefore giving more support in a lateral direction and in a front-rear direction. In addition, a lifting force is created, which facilitates lifting of the foot, since the resistance to movement or inertia is increased, when the supporting elements 21, 22 are moved closer to the leg. Since the ankle-foot orthosis 1 is tightened over ankles, when the concave middle section 34 is pressed down, the ankle-foot orthosis 1 is not loose, but sits tightly around the lower leg and foot. In some embodiments, the whole base plate element 36 has a concave shape, and thus in these embodiments the front and rear sections 32, 36 would not be flat.

Referring again to fig. 7, the intermediate portion 20 of the ankle-foot orthosis 1 comprises two substantially vertically oriented supporting elements 21, 22. The supporting elements 21, 22 are interconnecting the base plate element 30 and the upper portion 10. The inner supporting element 21 comprises a lower portion 211, an intermediate portion and an upper portion 210. The outer supporting element 22 comprises a lower portion 230, an intermediate portion 220 and an upper portion 210.

The top section 23 of the ankle-foot orthosis element 1 comprises an interconnecting part 231 that interconnects the two supporting elements 21, 22 at the end of their upper portions 210. The interconnecting part 231 is oriented towards the front of the wearer's lower limb, i.e. towards the tibia at the lower leg of the wearer during use. Optionally a damping pad is oriented and attached to the inside of the interconnecting part 23. The damping pad, if used provides comfort to the wearer and is made of an elastic material, such as a soft fabric or a foam rubber. The damping pad may be attached to the inside of interconnecting part 23 by means of an adhesive or other suitable fastening means such as a Velcro® tape for easy removal and cleaning of the pad. A fastening means, such as a strap, for fastening the ankle-foot orthosis to a lower part of a leg is also provided. A strap having a fold back strap portion can be fastened to any of the sides of interconnecting part 23 by putting it through at least one holder 24. The strap 13 is inserted into the holder 24, a performed fold back portion of the strap folds back onto the outside of the strap. Both the inside of a fold back strap portion and the outside of the strap may comprise hook and loop fasteners of Velcro® tape sections, such that the fold back portion conveniently can be fastened and loosened to and from the strap in order to giving the wearer access to the opening of strap or damping pad. The strap can be put through the holder 24 either from the rear or from the front, and thus it is possible to fasten and loosen the strap either on the left side or the right side of the ankle-foot orthosis. Alternatively, a buckle can be used for fastening the strap.

Straps can be made of a non-flexible material. This may provide for a durable strap. Straps can also be made in a flexible material, e.g. an elastic material and the straps can thereby provide for an increased motility and a decreased resistance. It may also provide for a more comfortable fit of the ankle-foot orthosis element to the leg.

According to an embodiment a method of manufacture of an ankle-foot orthosis element is provided. The method comprises providing of a substantially flat front section 32, a concave middle section 34, a substantially flat rear section 36, and thereby providing a base plate element 30. The method further comprises providing at least one supporting element 21, 22 in connection with the base plate element 30 and extending substantially vertically from the base plate element 30. Furthermore, the method comprises providing a top section 23 on top of the at least one supporting element 21, 22. The method also comprises provision of at least one holder 24 attached to the top portion 23. Provision of a limb fastening member arranged on the top section 23 for fastening the ankle-foot orthosis element 1 to a lower limb of a wearer is also comprised by the method.

Detailed manufacturing substeps may be derived from the structures as described in this specification, e.g. choice of material or fastening methods.

The ankle-foot orthosis 1 according to some of the embodiments as illustrated in the figures may be used by persons having foot ankle instabilities or difficulties to control the motion of the foot when walking. However, the ankle-foot orthosis 1 may also or instead be used during rehabilitation or preventative during sport activities.

The ankle-foot orthosis 1 may advantageously be made of one integral part and thus comprise the same material through-out the whole ankle-foot orthosis 1. Thereby manufacturing is simplified.

Preferably, the ankle-foot orthosis 1 is made of a lightweight material, such as a suitable plastic material that may be fiber-reinforced, e.g. with carbon or glass fibers or with talc, i.e. talcum powder. The material used for the ankle-foot orthosis may also be lighter, due to a lower density of the material. Other suitable materials comprise lightweight metals, such as titanium, magnesium, aluminum, or steel. Preferably, the ankle-foot orthosis is manufactured by injection moulding, but the lightweight materials may also be used in several layers or sections to provide favourable characteristics to the ankle-foot orthosis 1, including suitable combinations of the above-mentioned materials or an alloy thereof. This is to ensure that the ankle-foot orthosis 1 is as lightweight as possible in order to not hinder the wearer thereof, and at the same time to ensure sufficient structural strength for supporting the ankle or the foot or the lower limb of the wearer and to ensure flexibility of certain ankle-foot orthosis 1 sections. The lightweight material used should, preferably have a high structural strength. Injection moulding is a manufacturing process for producing parts from both thermoplastic and thermosetting plastic materials. During injection moulding, material is fed into a heated barrel, mixed, and forced into a mold cavity where it cools and hardens to the configuration of the cavity. Molds are made from metal, usually either steel or aluminum, and precision-machined to form the features of the desired part(s). Some advantages of injection molding are high production rates, repeatable high tolerances, the ability to use a wide range of materials, low labor cost, minimal scrap losses, and little need to finish parts after molding.

The ankle-foot orthosis 1 may also be made with a silver-reinforced material, which is a material, to which silver has been added. Silver is a material, which has bacteria-reducing properties. As an alternative, a copper-alloy-reinforced material may be used. A copper-alloy-reinforced material is a material, which has a copper-alloy added to it. Copper-alloys are known to have bacteria-reducing properties. Another suitable material for the ankle-foot orthosis 1 is polypropene, which is a tough, flexible and economical material, which has good resistance to fatigue. For sport applications, a soft material may be used.

Furthermore, the ankle-foot orthosis 1 may have a foot bed in a variety of standard sizes fitting most user's feet comfortably. The ankle-foot orthosis 1 may also be adapted to fit a wide variety of shoes, so that the user can use it together with his/her regular shoes. The ankle-foot orthosis element 1 further fits well together with insoles for shoes, such as sport shoes, which is important since the ankle-foot orthosis element 1 is intended to be placed between an insole of a shoe and the shoe or a foot bed thereof, so that the ankle-foot orthosis element 1 is placed in the shoe or the foot bed and thereafter the insole is placed on top of the base plate element 30 of the ankle-foot orthosis element 1.

The present disclosure has been described above with reference to specific embodiments. However, other embodiments than the above described are equally possible within the scope of the disclosure. Different method steps than those described above, may be provided within the scope of the disclosure. The different features and steps of the disclosure may be combined in other combinations than those described. The scope of the disclosure is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

## Claims

1. An ankle-foot orthosis element (1), comprising:
- a base plate element (30), comprising a substantially flat front section (32), a middle section (34), a substantially flat rear section (36);
- at least one supporting element (21, 22) supported by said base plate element (30);
- a top section (23) supported by said at least one supporting element (21, 22) and **characterised in that**
said base plate element (30) comprises a flexible concave shape at the middle section (34) of said base plate element (30) for fixating said ankle-foot orthosis element in a foot wear when flexed towards the foot wear.

2. The ankle-foot orthosis element (1) of claim 1, wherein said front section and said rear section of said base plate element (30) are substantially flat.

3. The ankle-foot orthosis element (1) according to claim 1 or 2, wherein the at least one supporting element (21, 22) are two supporting elements (21, 22) extending from said base plate element (30) to said top section (23) of said ankle-foot orthosis element (1),
wherein each of said supporting elements (21, 22) is extended in a substantially perpendicular direction to and away from said base plate, and
comprises at least one portion angled in a direction apart from the perpendicular direction of the extension of the supporting element from the base plate.

4. The ankle-foot orthosis element (1) according to claim 3, wherein said at least one portion is an intermediate portion (220).

5. The ankle-foot orthosis element (1) according to any of claims 1-4, further comprising a limb fastening member,
wherein the limb fastening member is arranged to said top section (23) for fastening said ankle-foot orthosis element (1) to a lower limb of a wearer, and wherein said limb fastening member optionally is a strap.

6. The ankle-foot orthosis element (1) according to any of the preceding claims, wherein said ankle-foot orthosis element (1) is one integral part.

7. The ankle-foot orthosis element (1) according to any of the preceding claims, wherein one of said at least one supporting elements (22) has an extended lower portion (230).

8. The ankle-foot orthosis element (1) according to claim 7, wherein said extended lower portion (230) of said one of said at least one supporting elements (22) is larger than and/or extending further towards the front section (32) than a lower portion (211) of another of said at least one supporting elements (21).

9. A foot wear, comprising an ankle-foot orthosis element (1) according to any of claims 1-8 integrated therein.

10. Use of an ankle-foot orthosis according to any of claims 1-8 for assisting persons subject to drop-foot and/or for supporting persons during sport activity and/or for supporting persons during rehabilitation and/or for prevention of injuries.

11. A method of manufacture of an ankle-foot orthosis element according to any of claims 1-8, comprising:
- providing a base plate element (30) comprising a front section (32), a flexible concave middle section (34) a rear section (36);
- providing at least one supporting element (21, 22) connectable with said base plate element (30) and extending substantially perpendicular to and away from said base plate element (30); and
- providing a top section (23) connectable to an opposite end of said at least one supporting elements (21, 22) connectable to the base plate, for better fit to the tibia.

12. The method according of claim 11, wherein said front and rear sections are made substantially flat.

13. The method according to claims 13 or 14, further comprising;
- providing a limb fastening member, such as a strap, arranged to said top section (23) for fastening said ankle-foot orthosis element (1) to a lower limb of a wearer.

14. The method of any of claims 11-13, wherein said ankle-foot orthosis element (1) is manufactured as one integral part.

15. The method of any of claims 11-14, wherein said ankle-foot orthosis element (1) is manufactured in several layers or sections, and/or is manufactured by injection moulding.

## Patentansprüche

1. Knöchel-Fuß-Orthose-Element (1), umfassend:
- Grundplatten-Element (30), welches einen im Wesentlichen flachen vorderen Abschnitt (32), einen mittleren Abschnitt (34), einen im Wesentlichen flachen hinteren Abschnitt (36) umfasst;
- mindestens ein Stützelement (21, 22), welches von dem Grundplatten-Element (30) gestützt wird;
- oberen Abschnitt (23), welcher von dem mindestens einen Stützelement (21,22) gestützt wird und welcher **dadurch gekennzeichnet ist, dass** das Grundplatten-Element (30) eine flexible konkave Form an dem mittleren Abschnitt (34) des Grundplatten-Elements (30) umfasst, welche zur Fixierung des Knöchel-Fuß-Orthose-Elements in einer Fußbekleidung dient, wenn es zur Fußbekleidung hin gebogen wird.

2. Knöchel-Fuß-Orthose-Element (1) gemäß Anspruch 1, wobei der vordere Abschnitt und der hintere Abschnitt des Grundplatten-Elements (30) im Wesentlichen flach sind.

3. Knöchel-Fuß-Orthose-Element (1) gemäß Anspruch 1 oder 2, wobei es sich bei dem mindestens einen Stützelement (21,22) um zwei Stützelemente (21, 22) handelt, die sich von dem Grundplatten-Element (30 zu dem oberen Abschnitt (23) des Knöchel-Fuß-Orthose-Elements (1) hin erstrecken,
wobei sich jedes der Stützelemente (21, 22) in einer im Wesentlichen senkrechten Richtung zu der Grundplatte hin und weg von dieser erstreckt, und
mindestens einen Teilabschnitt umfasst, der in eine Richtung abgesehen von der senkrechten Richtung der Erstreckung des Stützelements von der Grundplatte angewinkelt ist.

4. Knöchel-Fuß-Orthose-Element (1) gemäß Anspruch 3, wobei es sich bei dem mindestens einen Teilabschnitt um einen intermediären Teilabschnitt (220) handelt.

5. Knöchel-Fuß-Orthose-Element (1) gemäß einem beliebigen der Ansprüche 1 bis 4, ferner ein Extremitäten-Befestigungsbauteil umfassend,
wobei das Extremitäten-Befestigungsbauteil zum Zwecke der Befestigung des Knöchel-Fuß-Orthose-Elements (1) an einer unteren Extremität eines Trägers an dem oberen Abschnitt (23) angeordnet ist, und wobei es sich bei dem Extremitäten-Befestigungsbauteil optional um einen Riemen handelt.

6. Knöchel-Fuß-Orthose-Element (1) gemäß einem beliebigen der vorangehenden Ansprüche, wobei das Knöchel-Fuß-Orthose-Element (1) ein integrales Teil ist.

7. Knöchel-Fuß-Orthose-Element (1) gemäß einem beliebigen der vorangehenden Ansprüche, wobei eines der mindestens einen Stützelemente (22) einen erweiterten unteren Teilabschnitt (230) aufweist.

8. Knöchel-Fuß-Orthose-Element (1) gemäß Anspruch 7, wobei der erweiterte untere Teilabschnitt (230) des einen der mindestens einen Stützelemente (22) größer ist als und/oder sich weiter zu dem vorderen Abschnitt (32) hin erstreckt als ein unterer Teilabschnitt (211) eines anderen der mindestens einen Stützelemente (21).

9. Fußbekleidung, in welcher ein Knöchel-Fuß-Orthose-Element (1) gemäß einem beliebigen der Ansprüche 1 bis 8 integriert ist.

10. Verwendung eines Knöchel-Fuß-Orthose[-Elements] gemäß einem beliebigen der Ansprüche 1 bis 8 zur Unterstützung von Personen, die unter einem Fallfuß leiden, und/oder zur Unterstützung von Personen während sportlichen Aktivitäten und/oder zur Unterstützung von Personen während der Rehabilitation und/oder zur Vermeidung von Verletzungen.

11. Verfahren zur Herstellung eines Knöchel-Fuß-Orthose-Elements gemäß einem beliebigen der Ansprüche 1 bis 8, umfassend:
- Bereitstellung eines Grundplatten-Elements (30), welches einen vorderen Abschnitt (32), einen flexiblen konkaven mittleren Abschnitt (34), einen hinteren Abschnitt (36) umfasst;
- Bereitstellung mindestens eines Stützelements (21, 22), das sich mit dem Grundplatten-Element (30) verbinden lässt und sich im Wesentlichen senkrecht zu dem Grundplatten-Element (30) hin und weg von diesem erstreckt; und
- Bereitstellung eines oberen Abschnitts (23), der sich mit einem entgegengesetzten Ende des mindestens eines Stützelements (21, 22) verbinden lässt, das sich mit der Grundplatte verbinden lässt, so dass es besser an das Schienbein passt.

12. Verfahren gemäß Anspruch 11, wobei der vordere und hintere Abschnitt im Wesentlichen flach ausgeführt sind.

13. Verfahren gemäß Ansprüchen 13 oder 14, ferner umfassend;
- Bereitstellung eines Extremitäten-Befestigungsbauteils, wie zum Beispiel eines Riemens, der an dem oberen Abschnitt (23) zum Zwecke der Befestigung des Knöchel-Fuß-Orthose-Elements (1) an einer unteren Extremität eines Trägers angeordnet ist.

14. Verfahren gemäß einem beliebigen der Ansprüche 11 bis 13, wobei das Knöchel-Fuß-Orthose-Element (1) als ein integrales Teil hergestellt ist.

15. Verfahren gemäß einem beliebigen der Ansprüche 11 bis 14, wobei das Knöchel-Fuß-Orthose-Element (1) in mehreren Schichten oder Abschnitten und/oder durch Spritzgießen hergestellt ist.

## Revendications

1. Elément d'orthèse pour la cheville et le pied (1), comprenant :
- un élément de plaque de base (30), comprenant une section avant substantiellement plate (32), une section du milieu (34), une section arrière substantiellement plate (36) ;
- au moins un élément de support (21, 22) supporté par ledit élément de plaque de base (30) ;
- une section supérieure (23) supportée par ledit au moins un élément de support (21, 22) et **caractérisé en ce que**
ledit élément de plaque de base (30) comprend une forme concave flexible au niveau de la section du milieu (34) dudit élément de plaque de base (30) pour une fixation dudit élément d'orthèse pour la cheville et le pied dans une chaussure lorsqu'il fléchit en direction de la chaussure.

2. Elément d'orthèse pour la cheville et le pied (1) selon la revendication 1, dans lequel ladite section avant et ladite section arrière dudit élément de plaque de base (30) sont substantiellement plates.

3. Elément d'orthèse pour la cheville et le pied (1) selon la revendication 1 ou 2, dans lequel le au moins un élément de support (21, 22) consiste en deux éléments de support (21, 22) s'étendant à partir dudit élément de plaque de base (30) vers ladite section supérieure (23) dudit élément d'orthèse pour la cheville et le pied (1),
dans lequel chacun desdits éléments de support (21, 22) s'étendent dans une direction substantiellement perpendiculaire se rapprochant et s'écartant de ladite plaque de base, et
comprend au moins une partie inclinée dans une direction s'écartant de la direction perpendiculaire à l'extension de l'élément de support par rapport à la plaque de base.

4. Elément d'orthèse pour la cheville et le pied (1) selon la revendication 3, dans lequel ladite au moins une partie est une partie intermédiaire (220).

5. Elément d'orthèse pour la cheville et le pied (1) selon l'une quelconque des revendications 1 à 4, comprenant en outre un élément de fixation de membre, dans lequel l'élément de fixation de membre est disposé sur ladite section supérieure (23) pour une fixation dudit élément d'orthèse pour la cheville et le pied à un membre inférieur d'un porteur, et dans lequel ledit élément de fixation de membre est optionnellement une bande.

6. Elément d'orthèse pour la cheville et le pied (1) selon l'une quelconque des revendications précédentes, dans lequel ledit élément d'orthèse pour la cheville et le pied (1) est une seule pièce.

7. Elément d'orthèse pour la cheville et le pied (1) selon l'une quelconque des revendications précédentes, dans lequel un dudit au moins un élément de support (22) a une partie inférieure étendue (230).

8. Elément d'orthèse pour la cheville et le pied (1) selon la revendication 7, dans lequel ladite partie inférieure étendue (230) dudit un dudit au moins un élément de support (22) est plus grand et/ou s'étend plus loin en direction de la section avant (32) qu'une partie inférieure (211) d'un autre dudit au moins un élément de support (21).

9. Chaussure, comprenant un élément d'orthèse pour la cheville et le pied (1) selon l'une quelconque des revendications 1 à 8 intégré dans celle-ci.

10. Utilisation d'une orthèse pour la cheville et le pied selon l'une quelconque des revendications 1 à 8 pour aider des personnes sujettes à un pied tombant et/ou pour aider des personnes au cours d'une activité sportive et/ou pour aider des personnes au cours d'une convalescence et/ou pour prévenir des blessures.

11. Procédé de fabrication d'un élément d'orthèse pour la cheville et le pied selon l'une quelconque des revendications 1 à 8, comprenant les étapes consistant à :
- fournir un élément de plaque de base (30) comprenant une section avant (32), une section du milieu concave flexible (34), une section arrière (36) ;
- fournir au moins un élément de support (21, 22) pouvant être connecté audit élément de plaque de base (30) et s'étendant substantiellement perpendiculairement en se rapprochant et en s'écartant dudit élément de plaque de base (30) ; et
- fournir une section supérieure (23) pouvant être connectée à une extrémité opposée dudit au moins un élément de support (21, 22) pouvant être connecté à la plaque de base, pour mieux s'ajuster sur le tibia.

12. Procédé selon la revendication 11, dans lequel lesdites sections avant et arrière sont réalisées en étant substantiellement plates.

13. Procédé selon la revendication 13 ou 14, comprenant en outre l'étape consistant à :
- fournir un élément de fixation de membre, tel qu'une bande, disposé sur ladite section supérieure (23) pour fixer ledit élément d'orthèse pour la cheville et le pied (1) sur un membre intérieur d'un porteur.

14. Procédé selon l'une quelconque des revendications 11 à 13, dans lequel ledit élément d'orthèse pour la cheville et le pied (1) est fabriqué en une seule pièce.

15. Procédé selon l'une quelconque des revendications 11 à 14, dans lequel ledit élément d'orthèse pour la cheville et le pied (1) est fabriqué en plusieurs couches ou sections, et/ou est fabriqué par moulage par injection.
